# EUROPEAN PATENT APPLICATION

(11) **EP 4 613 850 A1**
(43) Date of publication of application: **10.09.2025**
(21) Application number: 23885750.2
(22) Date of filing: 31.10.2023
(51) Int. Cl.: C12N 5/0775, A61K 35/28, A61K 35/35, A61K 35/51, A61P 15/00, A61P 15/08

(54) **UTERINE TREATMENT COMPOSITION, UTERINE ENVIRONMENT-IMPROVING AGENT, IMPLANTATION AID, EMBRYO TRANSPLANTATION LIQUID, SPERM TRANSPLANTATION LIQUID, AND IMPLANTATION RATE-IMPROVING AGENT**

(30) Priority: 01.11.2022 JP 2022175839
(71) Applicant: Hiroshima University, Higashihiroshima-shi Hiroshima 739-8511 (JP); Rohto Pharmaceutical Co., Ltd., Osaka-shi, Osaka 544-8666 (JP)
(72) Inventor: SHIMADA Masayuki, Higashi-Hiroshima City, Hiroshima 7398511 (JP); UMEHARA Takashi, Higashi-Hiroshima City, Hiroshima 7398511 (JP); ISHIKAWA Noriyasu, Soraku-gun, Kyoto 619-0237 (JP); TAKIJIRI Takashi, Osaka-shi, Osaka 544-8666 (JP)
(74) Representative: Mewburn Ellis LLP
(86) International application number: PCT/JP2023/039207
(87) International publication number: WO 2024/095996

(57) **Abstract**

The purpose of the present invention is to provide an excellent infertility treatment with which it is possible to increase the implantation rate of an embryo (fertilized egg) by improving the uterine environment. The present invention is a uterine treatment composition containing a mesenchymal stem cell culture supernatant, and is used as a uterine environment-improving agent, an implantation aid, an embryo transplant liquid, a sperm transplantation liquid, or an implantation rate-improving agent. The mesenchymal stem cells are preferably derived from adipose tissue, umbilical cord tissue, or bone marrow tissue.

## Description

### TECHNICAL FIELD

The present invention relates to a composition for treating the uterus, an agent for improving uterine environment, an agent for aiding implantation, an embryo transfer solution, a sperm transfer solution, and an agent for improving implantation rate.

### BACKGROUND ART

"Infertility" refers to the inability to conceive for a certain period despite unprotected sexual intercourse between a healthy man and woman who wish to conceive, and this "certain period " is defined as "generally one year" by the Japan Society of Obstetrics and Gynecology. It is estimated that about one in ten couples are infertile, but in recent years, the age at which people think about conception has been rising, and it is known that both men and women become less likely to become pregnant as they age, and thus it is said that infertility rate is even higher.

Causes for infertility in the female include ovulation factors such as anovulation, fallopian tube factors such as the occlusion of the fallopian tube, the stenosis of the fallopian tube, and the adhesion of the fallopian tube, uterine factors such as uterine fibroid and endometrial polyp, cervical factors such as cervicitis and abnormality in mucus secretion from the uterine cervix, and immunological factors such as anti-sperm antibodies.

Poor endometrial blood flow, for example, due to uterine fibroid or congenital morphological abnormality of the uterus and the presence of adhesion or the like due to previous surgery or inflammation in the uterus interfere with the attachment and growth of an embryo that has reached the inside of the uterus, leading to difficulty in pregnancy.

As ovulation approaches, the mucus filling the uterine cervix changes, and the environment in the uterine cervix is conditioned for sperm to enter the uterus. Then, sperm reach the inside of the uterus, and acquire the ability to penetrate the zona pellucida on the surface of an ovum, but poor secretion of the mucus, the unsuitability of the mucus for the penetration of sperm, or the like complicates the arrival of sperm in the uterus, leading to difficulty in achieving fertilization, and thus in pregnancy.

In a female producing an anti-sperm antibody such as a sperm-immobilizing antibody because of some immunological abnormality, the antibody is also secreted into cervical mucus, and disadvantageously interrupts the passage of sperm, even if the sperm have good motility. The sperm-immobilizing antibody is also secreted in the fallopian tube, and even when sperm are injected deep into the uterine cavity in artificial insemination, the passage is disadvantageously interrupted in the fallopian tube. Therefore, also in the situation of fertilization, the sperm-immobilizing antibody may interfere with the binding between sperm and an ovum, leading to infertility.

Infertility treatment is carried out by selecting the optimal treatment method according to the cause. Major treatment methods include a timing method, an ovulation induction method, artificial insemination, and even assisted reproductive technology (ART) such as in vitro fertilization and microfertilization. The probability of giving birth by ART is said to be 11.7% on average in total treatment cases, and depends on age: the probability is approximately 20% under age of about 32 years, but decreases with age, and reaches 7 to 8% over age of 40 years; thus, in the current situation it cannot be said that the pregnancy rate is high. Fertility treatment causes heavy physical and mental burdens, and requires high cost as well. Accordingly, development of a novel fertility treatment is desired.

Mesenchymal stem cells are multipotent progenitor cells that were first isolated from bone marrow by Friedenstein (1982) (see Non Patent Document 1). It has been revealed that mesenchymal stem cells are present in various tissues such as bone marrow, umbilical cord, and fat, and mesenchymal stem cell transplantation is expected as a new therapeutic method for various intractable diseases (see Patent Documents 1 and 2). Recently, it has been known that cells having comparable functions are present in stromal cells in adipose tissue, placenta, umbilical cord, egg membrane, and the like. Therefore, mesenchymal stem cells are sometimes called mesenchymal stromal cells.

### PRIOR ART DOCUMENTS

### Patent Document

Patent Document 1: JP 2012-157263 A
Patent Document 2: JP 2012-508733 A

### Non Patent Document

Non Patent Document 1: Pittenger F. M. et al., Science 284, pp.143-147, 1999

### SUMMARY OF INVENTION

### Technical Problem

For improving the implantation rate, a technique called ERA test, which tests the endometrial implantation ability, is known. The ERA test is to find a timing of successful implantation by examining the gene expression profile of endometrial tissue, and not to improve the implantation rate itself. There are very few methods for improving the uterine environment for successful implantation, and development of a novel improving method has been demanded. Under the circumstances as described above, an object of the present invention is to provide excellent fertility treatment that can increase the implantation rate of embryos (fertilized ova) by improving the uterine environment.

### Solution to Problem

The present inventors have carried out intensive research in order to solve the above problem and as a result, found that a culture supernatant for mesenchymal stem (stromal) cells (MSCs) increases the implantation rate, and completed the present invention. According to the present invention, the implantation rate of embryos (fertilized ova) can be increased by improving the uterine environment. That is, the gist of the present invention is as follows.
[1] A composition for treating the uterus, comprising a mesenchymal stem cell culture supernatant.
[2] The composition for treating the uterus according to [1], wherein the composition is used as an agent for improving uterine environment, an agent for aiding implantation, an embryo transfer solution, a sperm transfer solution, or an agent for improving implantation rate.
[3] The composition for treating the uterus according to [1] or [2], wherein the mesenchymal stem cells are derived from adipose tissue, derived from umbilical cord tissue, or derived from bone marrow tissue.
[4] The composition for treating the uterus according to [1], wherein the mesenchymal stem cell culture supernatant is a mesenchymal stem cell culture supernatant obtained through culture of mesenchymal stem cells with a serum-free medium.

### Advantageous Effects of Invention

According to the present invention, the implantation rate of embryos (fertilized ova) can be increased by improving the uterine environment. For the uterus treated with the composition for treating the uterus according to the present invention, for example, promotion of decidualization and increase in the implantation rate of embryos (fertilized ova) in artificial insemination and in vitro fertilization can be expected.

### BRIEF DESCRIPTION OF DRAWINGS

[Fig. 1] Fig. 1 is a diagram showing the intrauterine vascular induction effect of a mesenchymal stem cell culture supernatant.
[Fig. 2] Fig. 2 is a diagram showing change caused by a mesenchymal stem cell culture supernatant in an endometrium (PAS staining (glycogen) and immunostaining of the cell growth marker Ki67).
[Fig. 3] Fig. 3 is a diagram showing change caused by a mesenchymal stem cell culture supernatant in an endometrium (HE staining).
[Fig. 4] Fig. 4 is a diagram showing change caused by a mesenchymal stem cell culture supernatant in an endometrium (PAS staining (glycogen)).
[Fig. 5] Fig. 5 is a diagram showing the morphology of an endometrium after transferring a fertilized ovum into the uterus treated with a mesenchymal stem cell culture supernatant.
[Fig. 6] Fig. 6 is a diagram showing the implantation rate (pregnancy rate)-increasing effect of a mesenchymal stem cell culture supernatant in transferring an in vitro fertilized ovum.
[Fig. 7] Fig. 7 is a diagram showing the proliferation promotion effect of a mesenchymal stem cell culture supernatant on endometrial epithelial cells.
[Fig. 8] Fig. 8 is a diagram showing that a mesenchymal stem cell culture supernatant exerts no proliferation promotion effect on endometrial stromal cells.
[Fig. 9-1] Fig. 9-1 is a diagram showing implantation rates in in vitro fertilization in Example 6.
[Fig. 9-2] Fig. 9-2 is a diagram showing pregnancy rates in in vitro fertilization in Example 6.

### DESCRIPTION OF EMBODIMENTS

Hereinafter, the composition for treating the uterus according to the present invention will be described in detail. In addition, the agent for improving uterine environment, the agent for aiding implantation, the embryo transfer solution, the sperm transfer solution, and the agent for improving implantation rate according to the present invention will be described.

### [Composition for treating the uterus]

The composition for treating the uterus according to the present invention is characterized by including a mesenchymal stem cell culture supernatant. The composition for treating the uterus according to the present invention can increase the implantation rate by improving the uterine environment by including a mesenchymal stem cell culture supernatant. For the uterus treated with the composition for treating the uterus according to the present invention, for example, promotion of decidualization and an increase in the implantation rate of embryos (fertilized ova) in artificial insemination and in vitro fertilization can be expected. The composition for treating the uterus according to the present invention may contain a further component as long as the further component does not impair the effect of the present invention, in addition to the mesenchymal stem cell culture supernatant as an essential component.

### (Mesenchymal stem cell culture supernatant)

In the present invention, the mesenchymal stem cell culture supernatant is a culture supernatant obtained through culture of mesenchymal stem cells.

In the present invention, the term "mesenchymal stem cells" refers to cells that have the ability to differentiate into one or more types of cells belonging to the mesenchymal lineages (such as osteocytes, cardiomyocytes, chondrocytes, tenocytes, adipocytes) and are capable of proliferating while maintaining that ability. The term "mesenchymal stem cells" used in the present invention refers to cells the same as stromal cells, and the two types of cells are not particularly distinguished from each other. The mesenchymal stem cells may be referred to as mesenchymal cells, simply. Examples of tissues containing mesenchymal stem cells include adipose tissue, umbilical cord, bone marrow, umbilical cord blood, endometrium, placenta, amnion, chorion, deciduum, dermis, skeletal muscle, periosteum, dental follicle, periodontium, dental pulp, and tooth germ. Examples of the mesenchymal stem cells in the present invention include those derived from adipose tissue, umbilical cord, bone marrow, umbilical cord blood, endometrium, placenta, amnion, chorion, deciduum, dermis, skeletal muscle, periosteum, dental follicle, periodontium, dental pulp, and tooth germ; adipose tissue-derived mesenchymal stem cells, umbilical cord-derived mesenchymal stem cells, and bone marrow-derived mesenchymal stem cells are preferred among them; and adipose tissue-derived mesenchymal stem cells and umbilical cord-derived mesenchymal stem cells are more preferred.

Examples of the species from which the mesenchymal stem cells in the present invention are derived include a human, a horse, cattle, a sheep, a pig, a dog, a cat, a rabbit, a mouse, and a rat.

The mesenchymal stem cells in the present invention may be derived from a species the same as or different from that of a subject to be treated (test subject).

The mesenchymal stem cells may be cells, for example, provided by any of PromoCell, Lonza, Biological Industries, Veritas, R&D Systems, and Corning, or cells prepared with a method well known to those skilled in the art. The mesenchymal stem cells may be primary cells separated from tissue of a donor, or cells of an established cell line.

The medium used for culturing the mesenchymal stem cells in the present invention is not particularly limited as long as the medium allows the mesenchymal stem cells to be cultured while maintaining them in good condition and is preferably a medium that allows human mesenchymal stem cells to proliferate while maintaining the ability to differentiate, for example, into osteocytes, chondrocytes, and adipocytes.

The medium used in the present invention may be prepared by supplementing a basal medium with a serum and/or with one or more serum substitutes such as albumin, transferrin, fatty acid, insulin, sodium selenite, cholesterol, a collagen precursor, a trace element, 2-mercaptoethanol, or 3'-thiolglycerol. In addition, if necessary, the medium may be further supplemented with a substance such as an amino acid such as glutamine, a sugar such as glucose, a metal salt such as sodium chloride or magnesium sulfate, a trace metal such as selenium, a lipid such as cholesterol or unsaturated fatty acid, a vitamin such as pantothenic acid, a protein such as albumin, insulin, transferrin, a growth factor, a proliferation factor, or a cytokine, a polysaccharide, a low-molecular-weight compound, an antibiotic, an antioxidant, a pyruvic acid, a buffer, or an inorganic salt.

Examples of the basal medium include IMDM medium, Medium 199 medium, Eagle's Minimum Essential Medium (EMEM) medium, αMEM medium, Dulbecco's modified Eagle's Medium (DMEM) medium, Ham's F12 medium, RPMI 1640 medium, Fischer 's medium, MCDB201 medium, and a mixed medium thereof.

From the viewpoint of use for treating the uterus, the medium that is used for culturing the mesenchymal stem cells for use in the present invention is preferably a xeno-free medium, which is free of a xeno-derived component such as a serum. Examples of such a medium include a medium provided as a pre-prepared medium for mesenchymal stem cells (stromal cells) such as Mesenchymal Stem Cell Growth Medium 2 (Ready-to-use, manufactured by PromoCell), Mesenchymal Stem Cell Growth Medium XF (Ready-to-use, manufactured by PromoCell), MSCGM BulletKittm, MSCGMtm Mesenchymal Stem Cell Growth Medium BulletKittm (manufactured by Lonza), a xeno-free medium for human mesenchymal stem cells (MSC NutriStem (registered trademark) XF, manufactured by Biological Industries), MesenCult-ACF Plus (manufactured by Veritas), StemXVivotm Serum-Free Human MSC Expansion Media (manufactured by R&D Systems, Corning), a serum-free medium for adipose-derived stem cells (KBM ADSC-4, manufactured by Kohjin Bio), and a serum-free medium for mesenchymal stem cells (R: STEM Medium for hMSC High Growth, manufactured by Rohto).

Examples of the serum described above include, but are not limited to, human serum, fetal bovine serum (FBS), bovine serum, calf serum, goat serum, horse serum, pig serum, sheep serum, rabbit serum, and rat serum. When a serum is used, a basal medium may be supplemented with 5 v/v% to 15 v/v%, preferably 10 v/v%, thereof.

Examples of the fatty acid include, but are not limited to, linoleic acid, oleic acid, linolenic acid, arachidonic acid, myristic acid, palmitoleic acid, palmitic acid, and stearic acid. Examples of the lipid include, but are not limited to, phosphatidylserine, phosphatidylethanolamine, and phosphatidylcholine. Examples of the amino acid include, but are not limited to, L-alanine, L-arginine, L-aspartic acid, L-asparagine, L-cysteine, L-cystine, L-glutamic acid, L-glutamine, and L-glycine. Examples of the protein include, but are not limited to, ecotin, reduced glutathione, fibronectin, and β2-microglobulin. Examples of the polysaccharide include, but are not limited to, a glycosaminoglycan, and particular examples of the glycosaminoglycan include, but are not limited to, hyaluronic acid and heparan sulfate. Examples of the proliferation factor include, but are not limited to, platelet-derived growth factor (PDGF), basic fibroblast growth factor (bFGF), transforming growth factor beta (TGF-β), hepatocyte growth factor (HGF), epidermal growth factor (EGF), connective tissue growth factor (CTGF), and vascular endothelial growth factor (VEGF).

### (Preparation of mesenchymal stem cell culture supernatant)

A supernatant for mesenchymal stem cells that is obtained with a method shown below can be used as the mesenchymal stem cell culture supernatant in the present invention. Alternatively, for example, a product obtained by removing unnecessary components from the supernatant by means of dialysis, ultrafiltration, or the like, a fraction obtained by fractionating the supernatant with a column or the like, a fraction selected with an antibody or the like against specific molecules, or a fraction obtained through a centrifugal operation may be used as the mesenchymal stem cell culture supernatant in the present invention.

A medium of the same type as the medium for culturing the mesenchymal stem cells can be used as the medium that is used in obtaining the culture supernatant. The method for obtaining the culture supernatant is not particularly limited as long as the method is suitable for culture of the respective mesenchymal stem cells, and an example is a method in which mesenchymal stem cells are cultured at a temperature of 20°C to 37°C under a 2% to 7% CO₂ environment and a 5% to 21% O₂ environment, preferably at room temperature to 37°C under a 5% CO₂ environment, and the culture supernatant is obtained.

The mesenchymal stem cell culture supernatant according to the present invention needs contact between the mesenchymal stem cells and the medium, and even a washing solution resulting from washing the mesenchymal stem cells with the medium can be used for the culture supernatant in the present invention. The time of contact between the mesenchymal stem cells and the medium is, for example, 14 days or less, preferably 10 days or less, further preferably 7 days or less, and more preferably 5 days or less. The culture to obtain the culture supernatant may be plate culture with adhesion to a flask, or suspension/stirring culture with adhesion to microbeads or the like.

The amount of the mesenchymal stem cell culture supernatant included in the composition for treating the uterus according to the present invention is 0.1% by weight to 100% by weight, preferably 0.5% by weight to 95% by weight, more preferably 1% by weight to 90% by weight, further preferably 3% by weight to 80% by weight, and particularly preferably 5% by weight to 50% by weight, of the whole composition for treating the uterus. By setting the amount of the mesenchymal stem cell culture supernatant included in the composition for treating the uterus according to the present invention within the above numerical range, the composition for treating the uterus according to the present invention has an excellent effect of increasing the implantation rate by improving the uterine environment.

The composition for treating the uterus according to the present invention may contain a further component as long as the further component does not impair the effect of the present invention, in addition to the mesenchymal stem cell culture supernatant. Examples of the further component include a protective agent such as dimethylsulfoxide (DMSO) or serum albumin, an antibiotic, a vitamin, a carrier, an excipient, a disintegrant, a buffer, an emulsifier, a stabilizer, a preservative, an antiseptic, and physiological saline.

The species that is the target of the composition for treating the uterus according to the present invention may be any mammalian animal, and examples thereof include a human, a horse, cattle, a sheep, a pig, a dog, a cat, a rabbit, a mouse, a rat, and a rare animal.

The composition for treating the uterus according to the present invention is used as a liquid that comes into direct contact with the uterus such as an agent for aiding implantation, an embryo transfer solution, an agent for improving uterine environment, a sperm transfer solution, or an agent for improving implantation rate.

The agent for aiding implantation is a formulation for assisting in the successful occurrence of "implantation", a phenomenon in which a fertilized ovum comes into contact with an endometrium and settles thereon. The embryo transfer solution is a solution for housing an embryo (fertilized ovum) therein in performing "embryo transfer", an operation to grow an embryo (fertilized ovum) to some extent and then return it to the uterus. The agent for improving uterine environment is a formulation that is used for improving the environment in the uterus to a state more suitable for pregnancy. The agent for improving uterine environment according to the present invention can be used for artificial insemination or in vitro fertilization (increased success rate or efficiency), breeding of livestock (increased success rate or efficiency of artificial insemination), breeding or maintenance of species (for example, maintenance of endangered species, or maintenance or crossbreeding of a pet strain), or treatment or improvement of various diseases caused mainly or secondarily by uterine disorder (for example, uterine disorder after malignant tumor surgery or chemotherapy), and the like. The sperm transfer solution is a solution for suspending sperm therein in injecting sperm into a uterus in artificial insemination. The agent for improving implantation rate is a formulation for increasing the implantation rate of embryos (fertilized ova).

The composition for treating the uterus according to the present invention is capable of inducing decidualization by acting on the uterus to elevate expression of implantation-related genes in endometrial epithelial cells. Examples of the implantation-related genes include Lif (Leukemia Inhibitory Factor), FOXO1 (Forkhead box protein O1), Hoxa-10 (Homeobox A10), and Integrin beta-3 (Integrin β3, Int3b).

The composition for treating the uterus according to the present invention can be prepared by a conventional method after mixing a further necessary component with the mesenchymal stem cell culture supernatant described above.

### EXAMPLES

Hereinafter, the present invention will be described in detail with reference to Examples and Test Examples, but the present invention is not limited by these Examples and the like.

### [Example 1: Preparation of culture supernatant and detection of components contained in culture supernatant]

Umbilical cord mesenchymal stem cells (C-12971 Human Mesenchymal Stem Cells from Umbilical Cord Matrix (hMSC-UC), manufactured by PromoCell) were cultured in RIM medium (serum-free medium for mesenchymal stem cells, containing EGF, bFGF, albumin, transferrin, and insulin, Rohto) or RS medium (serum-free medium for mesenchymal stem cells, containing EGF, bFGF, albumin, transferrin, and insulin, Rohto) for 3 days, and culture supernatants were obtained (hereinafter referred to as "3D RIM culture supernatant" and "3D RS culture supernatant"). In addition, culture was performed for 3 days, the medium was then exchanged, culture was further performed for 1 day, and culture supernatants were obtained (hereinafter referred to as "1D RIM culture supernatant" and "1D RS culture supernatant"). Factors contained in the culture supernatants were determined by ELISA, and it was confirmed that PGE2, IL-6, HGF, MCP1, MMP2, BDNF, and CD63+ exosomes were contained in any of them.

### [Example 2: Vascular induction]

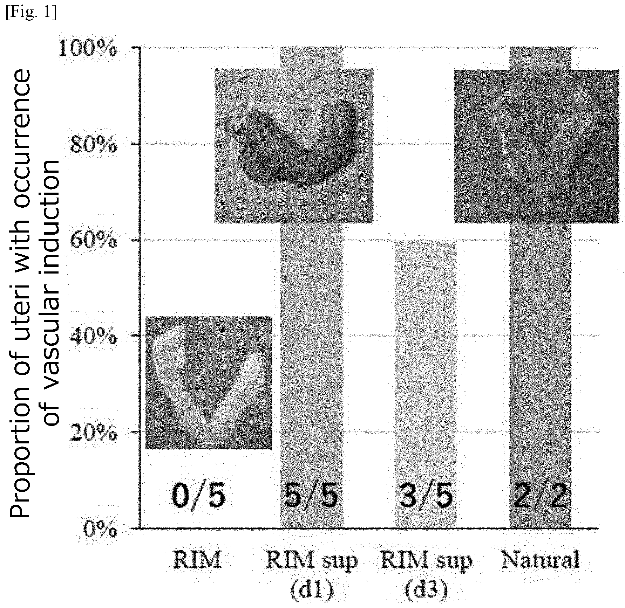

HTF Medium (manufactured by FUJIFILM Wako Pure Chemical Corporation) supplemented with the 3D RIM culture supernatant or the 1D RIM culture supernatant in such a way as to have a final concentration of 10% or HTF Medium supplemented with no supernatant as a negative control (control) was injected into the uterus of an 8-week-old female mouse. As a positive control, an 8-week-old female mouse was mated with a 12-week-old male mouse. Two days after the injection of them, the uterus was excised; the images are shown in Fig. 1. In addition, the proportion of uteri with the occurrence of vascular induction under each condition is represented as a graph.

As shown in Fig. 1, it was confirmed that the supplementation with the cell culture supernatant induced blood vessels and thus induced the same uterine change as caused by coital stimulation.

### [Example 3: Change in endometrium (improvement of uterine environment)]

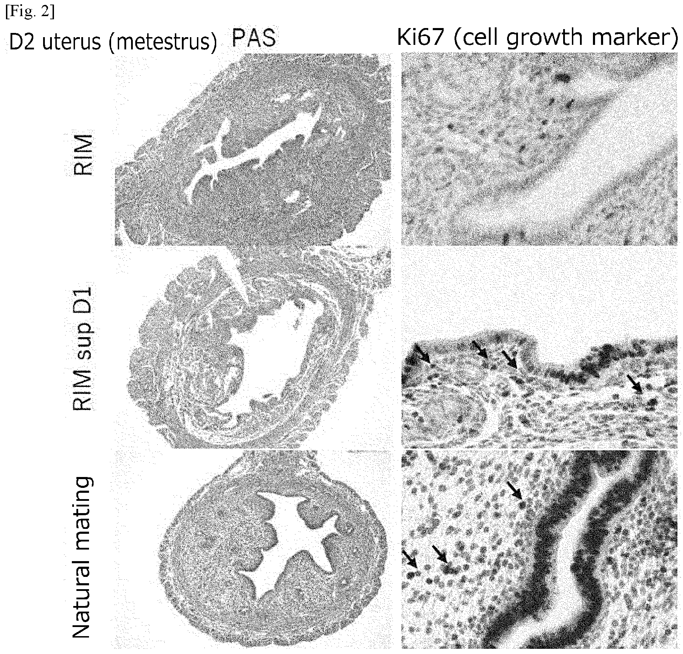

HTF Medium (manufactured by FUJIFILM Wako Pure Chemical Corporation) supplemented with the 1D RIM culture supernatant in such a way as to have a final concentration of 10% or HTF Medium supplemented with no supernatant as a negative control (control) was injected into the uterus of an 8-week-old female mouse. As a positive control, an 8-week-old female mouse was mated with a 12-week-old male mouse. Two days after the injection of them, the uterus was excised, fixed with 4% paraformaldehyde for 12 hours, and then embedded in paraffin. The embedded uterus was processed into sections, which were tested by PAS staining to stain glycogen and immunostaining with an antibody capable of recognizing the cell growth marker Ki67. The staining images are shown in Fig. 2.

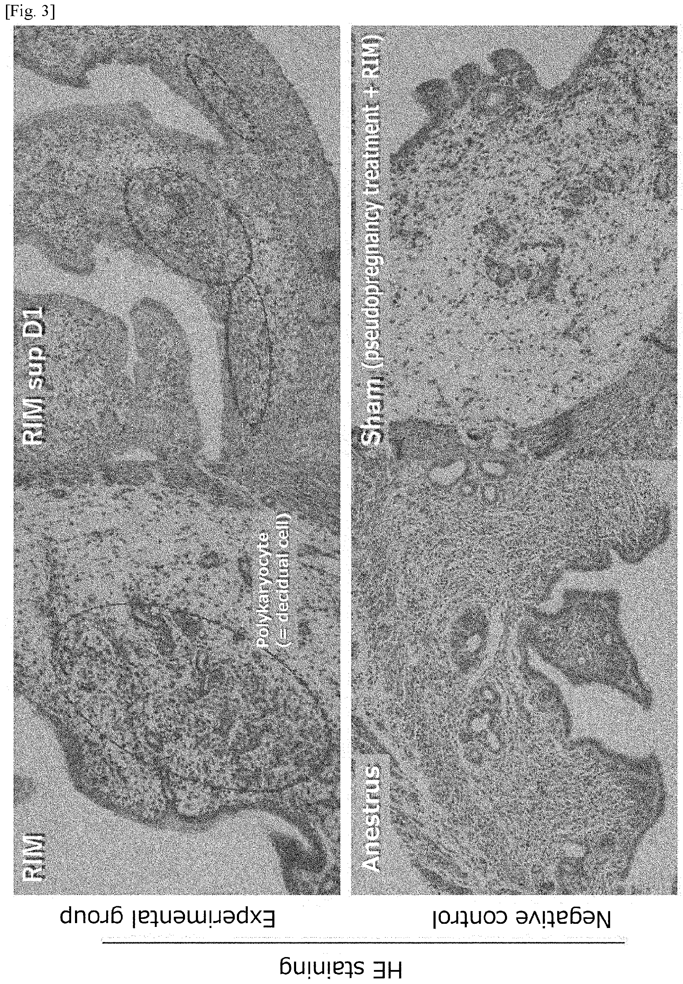

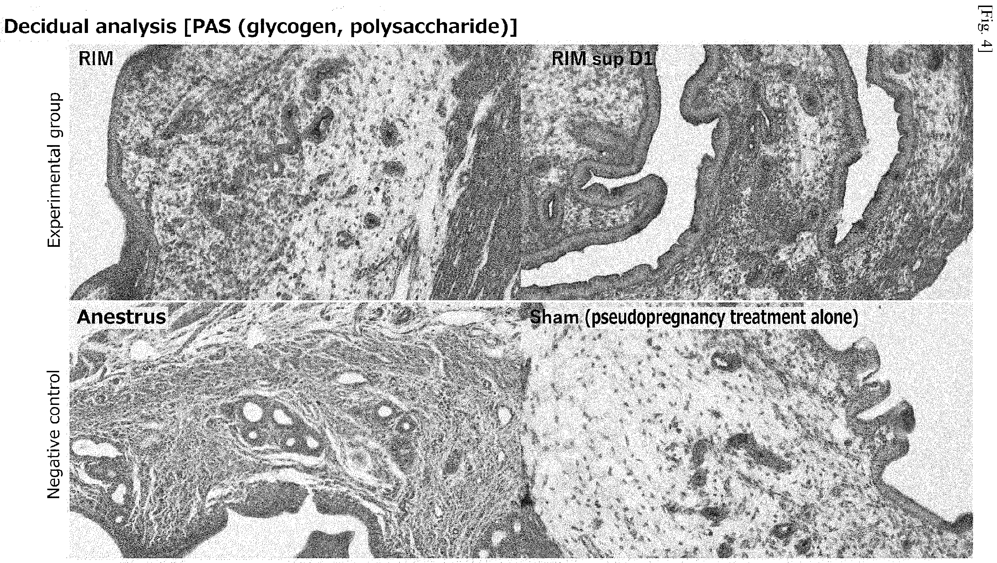

HTF Medium (manufactured by FUJIFILM Wako Pure Chemical Corporation) supplemented with the 1D RIM culture supernatant in such a way as to have a final concentration of 10% or HTF Medium supplemented with no supernatant as a negative control (control) was injected into the uterus of an 8-week-old female mouse. Four days thereafter, cone oil was surgically injected to induce the decidualization of the endometrium. Three days after the injection of cone oil, the uterus was excised, fixed with 4% paraformaldehyde for 12 hours, and then embedded in paraffin. The embedded uterus was processed into sections, which were subjected to HE staining for observation of morphology, and subjected to PAS staining for examining the glycogen accumulation. The HE staining images are shown in Fig. 3, and the PAS staining images are shown in Fig. 4.

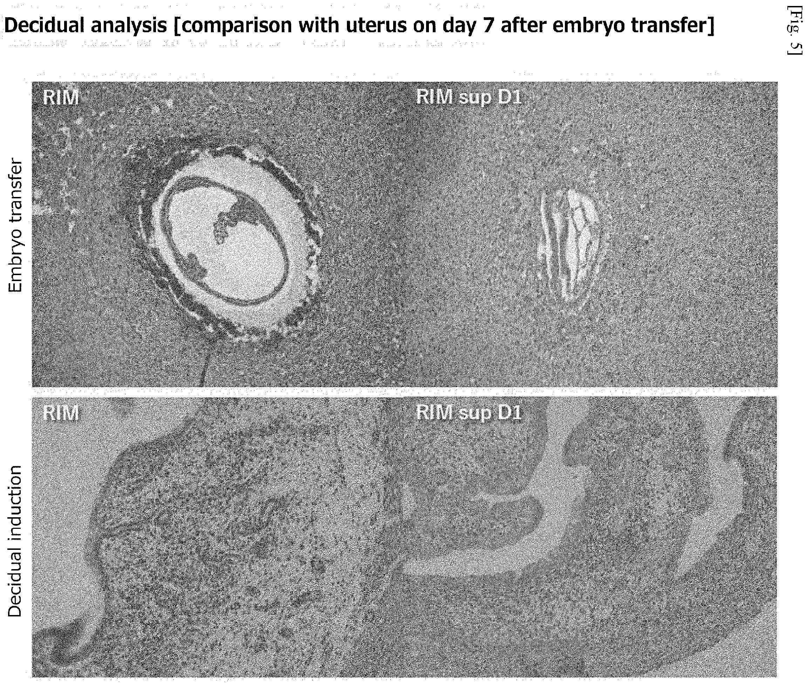

In vitro fertilization was performed by using an ovum collected from a 3-week-old female mouse and sperm collected from a 12-week-old male mouse to create an in vitro fertilized ovum. HTF Medium (manufactured by FUJIFILM Wako Pure Chemical Corporation) supplemented with the 1D RIM culture supernatant in such a way as to have a final concentration of 10% or HTF Medium supplemented with no supernatant as a negative control (control) was injected into the uterus of an 8-week-old female mouse, and the next day the in vitro fertilized ovum created above was transferred into the fallopian tube. Seven days thereafter, the uterus was excised, fixed with 4% paraformaldehyde for 12 hours, and then embedded in paraffin. The embedded uteri were processed into sections, and the morphologies were observed by HE staining. The staining images are shown in Fig. 5.

As shown in Figs. 2 to 5, it was confirmed that the injection of the cell culture supernatant into the uterus caused cell proliferation through accumulation of glycogen followed by catabolism of glycogen, that is, the cell culture supernatant was capable of improving the uterine environment by promoting decidualization to cause change into an endometrium ready for successful implantation.

### [Example 4: In vitro fertilization - 1]

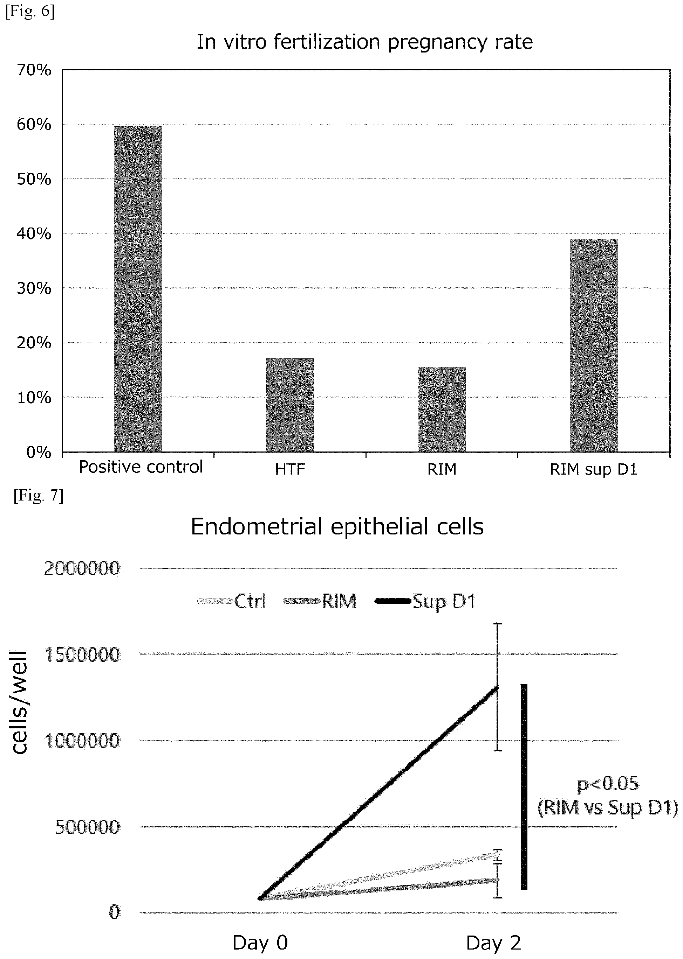

In vitro fertilization was performed by using an ovum collected from a 3-week-old female mouse and sperm collected from a 12-week-old male mouse to create an in vitro fertilized ovum. HTF Medium (manufactured by FUJIFILM Wako Pure Chemical Corporation) supplemented with the 1D RIM culture supernatant in such a way as to have a final concentration of 10% or HTF Medium supplemented with no supernatant as a negative control (control) was injected into the uterus of an 8-week-old female mouse, and the next day 20 of the created in vitro fertilized ova were transferred into the fallopian tube. Seven days thereafter, Evans Blue solution was injected from the tail vein of each mouse, the uteri were then excised, and the number of implantation was counted. In addition, the implantation rates (pregnancy rates) were determined; the results are shown in Fig. 6.

As shown in Fig. 6, it was confirmed that the implantation rate (pregnancy rate) of fertilized ova from in vitro fertilization was increased by treating the inside of the uterus with the cell culture supernatant.

### [Example 5: In vitro culture of endometrium stromal cells]

From a 12-week-old female C57BL6 mouse to which estradiol had been administered to induce estrus, the uterus was excised. The excised uterus was incised, and then treated with HBSS solution (CaCl₂·2H₂O 0.185 g/L, MgSO₄ 0.098 g/L, KCl 0.400 g/L, KH₂PO₄ 0.060 g/L, NaHCO₃ 0.350 g/L, NaCl 8.000 g/L, Na₂HPO₄ 0.048 g/L, D-Glucose 1.0 g/L) containing 25 mg/mL pancreatin (P1750; Sigma-Aldrich) and 0.25% (v/v) trypsin EDTA (NACALAI TESQUE, INC.) to isolate epithelial cells. The cells in suspension were seeded on a collagen-coated dish (manufactured by IWAKI), and then treated with HBSS containing 0.1 mg/mL collagenase type I (Worthington) and 0.05% (v/v) trypsin EDTA to isolate stromal cells. These cells were seeded on a collagen-coated dish, and cultured with modified DMEM and Ham's F-12 medium (10% fetal bovine serum, 100 U/mL penicillin, 100 µg/mL streptomycin) containing 10% 1D RIM culture supernatant or modified DMEM and Ham's F-12 medium containing no culture supernatant as a control at 37°C, 5% CO₂, and 95% atmosphere under a humid environment for 48 hours.

After washing with HBSS, the cells were peeled off with 0.25% (v/v) trypsin, and the number of cells was counted with an Automated Cell Counter TC20TM.

The oxygen consumption rate (OCR) and extracellular oxidation rate (ECAR) of cultured cells were measured with an extracellular flux analyzer (XF HS Mini; Agilent Technologies). The cultured cells were peeled off, then suspended in RPMI medium (manufactured by Agilent Technologies, supplemented with D-Glucose 10 mM, pyruvic acid 1 mM, L-Glutamine 2 mM, and 1% FBS), and seeded on wells for analysis.

In extracellular flux analysis, measurement of ECAR was performed for five cycle, D-Glucose (final concentration: 10 mM; Agilent Technologies) was then injected, and measurement was performed for three cycles, wherein each cycle was a 9-minute operation (Mix 3 min/Wait 3 min/Measure 3 min). A value obtained in measurement at the fifth cycle was used as Basal ECAR. Glycolysis was calculated by subtracting the value of Basal ECAR from a measurement at the eighth cycle.

Measurement of OCR was performed as follows:
(1) RPMI medium alone for five cycles [initial OCR];
(2) ATP synthesis inhibition with Oligomycin (Sigma-Aldrich) at a final concentration of 1 µM for three cycles [post-oligomycin OCR];
(3) mitochondrial uncoupling with carbonyl cyanide 4-(trifluoromethoxy)phenylhydrazone (FCCP; Sigma-Aldrich) at a final concentration of 5 µM for three cycles [post-FCCP OCR]; and
(4) inhibition of the electron transport chain with Rotenone (Sigma-Aldrich)/Antimycin A (Sigma-Aldrich) at final concentrations of 1 µM for three cycles [post-rotenone/antimycin-A OCR]. Values at the final cycles in the respective conditions (values at 5th, 8th, 11th, and 14th cycles) were used for calculation of measurements of OCR.

The indexes were calculated according to the following procedures:
Basal OCR = initial OCR - post-rotenone/antimycin-A OCR
ATP Production = Basal OCR - post-oligomycin OCR

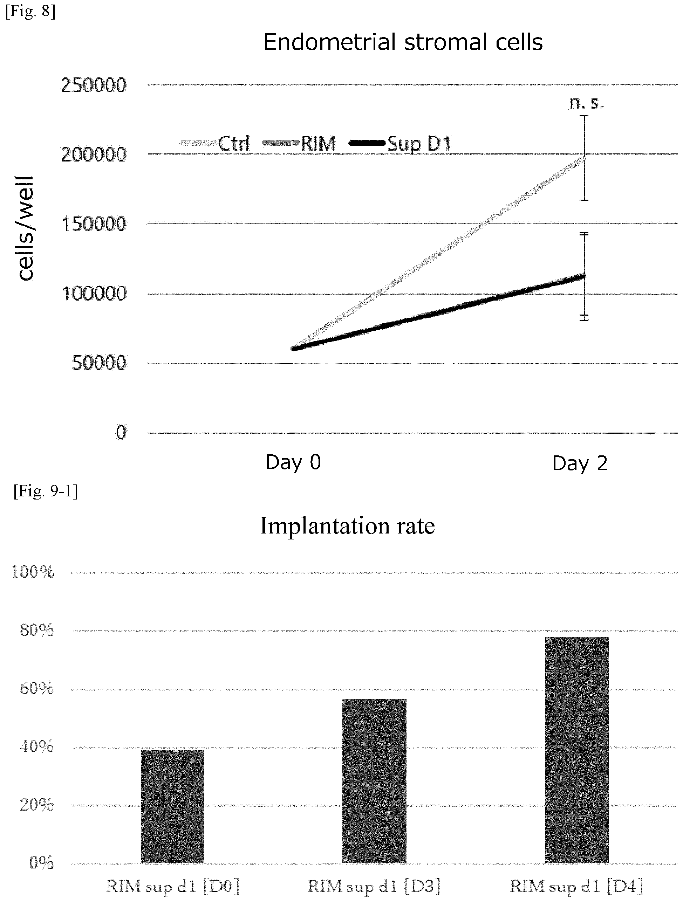

As shown in Fig. 7, the supplementation with the supernatant significantly promoted the proliferation of endometrial epithelial cells. By contrast, the proliferation promotion effect by the supplementation with the supernatant was not obtained in endometrial stromal cells (Fig. 8), and the action was selective for endometrial epithelial cells.

### [Example 6: In vitro fertilization - 2]

Umbilical cord mesenchymal stem cells (C-12971 Human Mesenchymal Stem Cells from Umbilical Cord Matrix (hMSC-UC), manufactured by PromoCell) were cultured in RIM medium (Certificate for Consultation on Material Eligibility of Regenerative, Cellular Therapy ad Gene Therapy Products and application for registration for Master File for Drug Substances, etc., have been scheduled; a serum-free medium for mesenchymal stem cells, containing EGF, bFGF, albumin, transferrin, and insulin, Rohto) for 3 days, the medium was then exchanged, further cultured for 1 day, and culture supernatants were obtained (hereinafter referred to as "1D RIM culture supernatant"). Factors contained in the culture supernatants were determined by ELISA, and it was confirmed that PGE2, IL-6, HGF, MCP1, MMP2, BDNF, and CD63+ exosomes were contained in any of them.

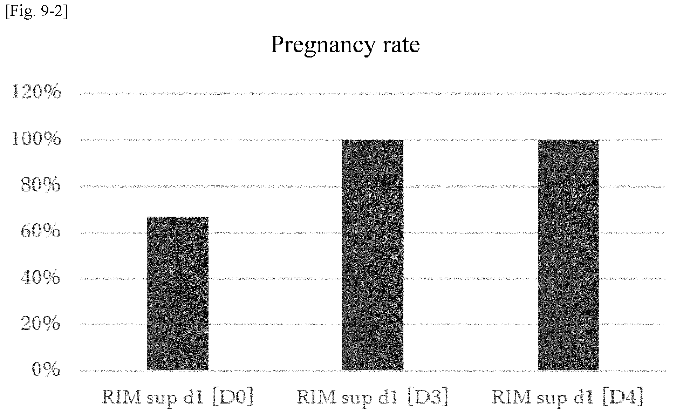

In vitro fertilization was performed by using an ovum collected from a 3-week-old female mouse and sperm collected from a 12-week-old male mouse to create an in vitro fertilized ovum. The created in vitro fertilized ovum was transferred into the fallopian tube of an 8-week-old female mouse, and HTF Medium (manufactured by FUJIFILM Wako Pure Chemical Corporation) supplemented with the 1D RIM culture supernatant in such a way as to have a final concentration of 10% was injected into the uterus of the 8-week-old female mouse on day 0, 3, and 4 as the day of the transfer of the in vitro fertilized ovum was defined as day 1. Seven days thereafter, Evans Blue solution was injected from the tail vein of each mouse, the uterus was then excised, and the number of implantation was counted. The implantation rates and pregnancy rates were determined; the results are shown in Fig. 9-1 (implantation rates) and Fig. 9-2 (pregnancy rates).

As shown in Figs. 9-1 and 9-2, it was confirmed that the implantation rate (pregnancy rate) of fertilized ova from in vitro fertilization was increased by treating the inside of the uterus with the cell culture supernatant.

Elevated expression of implantation-related genes (Lif, FOXO1, Hoxa-10, Int3b) in endometrial epithelial cells was found in the group with HTF Medium supplemented with the 1D RIM culture supernatant, suggesting the possibility that preparation for implantation was promoted in the endometrial epithelial cells. Further, nuclear import of FOXO1 was found, and this is inferred to indicate progressive induction of decidualization. The 1D RIM culture supernatant was revealed to have effects of improving the uterine environment, assisting in implantation of fertilized ova, and improving the implantation rate of fertilized ova. The 1D RIM culture supernatant can be preferably used for an agent for improving uterine environment, an agent for aiding implantation, an embryo transfer solution, a sperm transfer solution, or an agent for improving implantation rate.

### INDUSTRIAL APPLICABILITY

The composition for treating the uterus according to the present invention can be used for artificial insemination or in vitro fertilization (increased success rate or efficiency), breeding of livestock (increased success rate or efficiency of artificial insemination), breeding or maintenance of species (for example, maintenance of endangered species, or maintenance or crossbreeding of a pet strain), or treatment or improvement of various diseases caused mainly or secondarily by uterine disorders (for example, uterine disorder after malignant tumor surgery or chemotherapy), and the like.

## Claims

1. A composition for treating a uterus, comprising a mesenchymal stem cell culture supernatant.

2. The composition for treating a uterus according to claim 1, wherein the composition is used as an agent for improving uterine environment, an agent for aiding implantation, an embryo transfer solution, a sperm transfer solution, or an agent for improving implantation rate.

3. The composition for treating a uterus according to claim 1 or 2, wherein the mesenchymal stem cells are derived from adipose tissue, derived from umbilical cord tissue, or derived from bone marrow tissue.

4. The composition for treating a uterus according to claim 1, wherein the mesenchymal stem cell culture supernatant is a mesenchymal stem cell culture supernatant obtained through culture of mesenchymal stem cells with a serum-free medium.
